Europäisches Patentamt

European Patent Office

Office européen des brevets

(19)

(11) **EP 0 453 330 B2**

(12) **NOUVEAU FASCICULE DE BREVET EUROPEEN**

(45) Date de publication et mention de la décision concernant l'opposition:
**03.12.1997 Bulletin 1997/49**

(45) Mention de la délivrance du brevet:
**03.02.1993 Bulletin 1993/05**

(21) Numéro de dépôt: **91400586.3**

(22) Date de dépôt: **04.03.1991**

(51) Int. Cl.$^6$: **C07B 57/00**, C07D 303/48

(54) **Procédé de préparation du (-) (2R, 3S)-2,3-époxy-3-(4-méthoxyphényl)propionate de méthyle et de son énantiomère**

Verfahren zur Herstellung von (-) (2R,3S)-2,3-Epoxy-3-(4-methoxyphenyl)-propionsäure-methylester und dessen Enantiomer

Process for the preparation of (-) (2R,3S)-2,3-epoxy-3-(4-methoxyphenyl) propionic acid methylester and its enantiomer

(84) Etats contractants désignés:
**AT BE CH DE DK ES FR GB GR IT LI LU NL SE**

(30) Priorité: **15.03.1990 FR 9003310**

(43) Date de publication de la demande:
**23.10.1991 Bulletin 1991/43**

(73) Titulaire: **SYNTHELABO**
**92350 Le Plessis Robinson (FR)**

(72) Inventeurs:
- **Zard, Lydia**
  **La Croix Audierne, F-91190 Gif S/Yvette (FR)**

- **Tixidre Arlette**
  **F-91400 Orsay (FR)**
- **Rossey, Guy**
  **F-78960 Voisins le Bretonneux (FR)**
- **Wick, Alexander**
  **F-78860 St Nom La Breteche (FR)**

(74) Mandataire: **Ludwig, Jacques et al**
**SYNTHELABO,**
**Service Brevets,**
**B.P. 72**
**92352 Le Plessis Robinson Cédex (FR)**

(56) Documents cités:
**EP-A- 0 342 903**

Printed by Xerox (UK) Business Services
2.15.2/3.4

**Description**

La présente invertion a pour objet un procédé de préparation du (-)-(2R,3S)-2,3-époxy-3-(4-méthoxyphényl)propionate de méthyle par cristallisation préférentielle.

Le (-)-(2R,3S)-2,3-époxy-3-(4-méthoxyphényl)propionate de méthyle composé est un intermédiaire important dans la préparation de composés tels que le (+)-(2S,3S)-3-acétyloxy-2,3-dihydro-5-(2-diméthylaminoéthyl)-2-(4-méthoxyphényl)-1,5-5H-benzothiazépine-4-one, ou Diltiazem (DCI), et de ses dérivés.

Beaucoup d'efforts ont récemment été faits pour obtenir le (-)-(2R,3S)-2,3-époxy-3-(4-méthoxyphényl)propionate de méthyle, par dédoublement via un diastéréoisomère (demandes de brevets JP-13776/1985, JP-145174/1986 et EP-0342903), ou par hydrolyse enzymatique de l'énantiomère non voulu (demande de brevet EP-0343714). Ces procédés connus sont lourds et/ou de faible rendement, d'où on coût élevé du produit désiré.

Le procédé selon l'invention, au contraire, permet le dédoublement du racémate sans recourir aux méthodes mentionnées ci-dessus, en évitant donc leurs inconvénients.

On sait qu'un composé racémique peut exister sous deux formes cristallines dont l'une est un mélange racémique vrai, homogène, dont on ne peut pas séparer les énantiomères, et l'autre est appelée conglomérat. Les composés racémiques connus qui existent sous forme de conglomérat, c'est-à-dire sous forme d'un mélange d'énantiomères mécaniquement séparables, sont en nombre très limité (cf. *Enantiomers, Racemates and Resolutions*, de J. Jacques, A. Collet et S. H. Wilen, édité par John Wiley & Sons, 1981).

Les auteurs de la présente invention ont découvert que le trans (±)-2,3-époxy-3-(4-méthoxyphényl)propionate de méthyle peut exister, dans certaines conditions, sous forme de conglomérat.

On met en oeuvre le procédé de l'invention en mélangeant une solution saturée du *trans* (±)-2,3-époxy-3-(4-méthoxyphényl)propionate de méthyle avec une solution saturée de l'énantiomère lévogyre, et en recueillant par cristallisation une quantité supérieure dudit énantiomère.

Cela correspond à un dédoublement partiel ou total du compose racémique, dans les systèmes particuliers où ce composé existe en tant que conglomérat.

Conformément à l'invention on prépare, par exemple à la température de reflux du solvant, une solution saturée du composé racémique enrichie d'une quantité déterminée de l'énantiomère lévogyre. On ajoute à cette solution un germe cristallin de l'énantiomère en excès, on laisse cristalliser à une température et pendant un temps donnés, et on isole les cristaux formés.

Le *trans* (±)-2,3-époxy-3-(4-méthoxyphényl)propionate de méthyle peut être préparé par exemple à partir de p-anisaldéhyde et de chloroacétate de méthyle par une réaction de Darzens, comme décrit dans Chem. Pharm. Bull., 21, (12), 2786-2789 (1973), et le (-)-(2R,3S)-2,3-époxy-3-(4-méthoxyphényl)propionate de méthyle peut être préparé à partir du racémate par exemple comme décrit dans la demande de brevet JP-13776/1985 ou dans les demandes de brevets EP-0342903 et EP-0343714, déjà citées ci-dessus.

La solution de départ peut être obtenue à partir de *trans* (±)-2,3-époxy-3-(4-méthoxyphényl)propionate de méthyle et de (-)-(2R,3S)-2,3-époxy-3-(4-méthoxyphényl)propionate de méthyle. On dissout ces esters dans le t.-butylméthyléther.

La quantité d'ester optiquement pur à utiliser est comprise entre 0 et 5 parties, en poids, pour une partie d'ester racémique, et la concentration du mélange d'esters dans le solvant est de préférence comprise entre 2 et 50%.

La dissolution s'effectue à chaud, par exemple à la température de reflux du solvant et, lorsqu'elle est achevée, on ajoute à la solution, encore chaude ou revenue à température ambiante, un cristal d'ester optiquement pur, et on laisse refroidir le mélange, éventuellement en le plaçant dans une enceinte réfrigérée.

Il se produit alors la cristallisation préférentielle de l'énantiomère recherché, en excès donc par rapport à la quantité mise en oeuvre au départ, et on isole ce dernier de manière connue, c'est-à-dire par filtration.

Le (-)-(2R,3S)-2,3-époxy-3-(4-méthoxyphényl)propionate de méthyle ainsi obtenu est pratiquement pur ; si on le désire, on peut encore le purifier par recristallisation, de manière connue.

Les exemples suivants illustrent en détail des modes de mise en oeuvre du procédé de l'invention.

Exemple 1

On dissout 5 g de *trans* (±)-2,3-époxy-3-(4-méthoxyphényl)propionate de méthyle racémique et 2 g de (-)-(2R,3S)-2,3-époxy-3-(4-méthoxyphényl)propionate de méthyle dans 100 ml de t.-butylméthyléther chauffé au reflux, tout en agitant. On arrête l'agitation, on ajoute à la solution chaude un cristal de (-)-(2R,3S)-2,3-époxy-3-(4-méthoxyphényl)propionate de méthyle, on laisse le mélange revenir à température ambiante, puis on le place à 5°C pendant 12 heures. On filtre les cristaux formés et on les sèche. On obtient ainsi 2,92 g de (-)-(2R,3S)-2,3-époxy-3-(4-méthoxyphényl)propionate de méthyle.
Point de fusion : 85°C.

Pureté* : ee > 95% (HPLC, colonne chirale Daicel® OD).
$[\alpha]_D^{20}$ = -199° (c = 1 ; MeOH).

Exemple 2

On dissout 1,5 g de *trans* (±)-2,3-époxy-3-(4-méthoxyphényl)propionate de méthyle racémique et 2 g de (-)-(2*R*,3*S*)-2,3-époxy-3-(4-méthoxyphényl)propionate de méthyle dans 15 ml de t.-butylméthyléther chauffé au reflux, sans agitation. A la solution obtenue on ajoute, à 50°C, un cristal de (-)-(2*R*,3*S*)-2,3-époxy-3-(4-méthoxyphényl)propionate de méthyle. On conserve le mélange à température ambiante pendant 1h15, puis on le place à 5°C pendant 5h. On filtre les cristaux formés, on les sèche, et on isole 2,23 g de (-)-(2*R*,3*S*)-2,3-époxy-3-(4-méthoxyphényl)propionate de méthyle.
Pureté : ee > 98%.

Exemple 3

On dissout 1,5 g de *trans* (±)-2,3-époxy-3-(4-méthoxyphényl)propionate de méthyle racémique et 3 g de (-)-(2*R*,3*S*)-2,3-époxy-3-(4-méthoxyphényl)propionate de méthyle dans 17 ml de t.-butylméthyléther chauffé au reflux.
A la solution obtenue on ajoute, à 22°C, un cristal de (-)-(2*R*,3*S*)-2,3-époxy-3-(4-méthoxyphényl)propionate de méthyle. On conserve le mélange à température ambiante pendant 30mn, puis on le place à 5°C pendant 2h30. On filtre les cristaux formés, on les sèche, et on isole 3,22 g de (-)-(2*R*,3*S*)-2,3-époxy-3-(4-méthoxyphényl)propionate de méthyle.

**Revendications**

1. Procédé de préparation du (-)-(2*R*,3*S*)-2,3-époxy-3-(4-méthoxyphényl)propionate de méthyle, consistant à préparer d'abord une solution saturée à partir de 1 partie, en poids, de *trans* (±)-2,3-époxy-3-(4-méthoxyphényl)propionate de méthyle et de 0 à 5 parties, en poids, de (-)-(2*R*,3*S*)-2,3-époxy-3-(4-méthoxyphényl)propionate de méthyle, puis à ajouter à la solution un germe cristallin de (-)-(2*R*,3*S*)-2,3-époxy-3-(4-méthoxyphényl)propionate de méthyle, à laisser cristalliser et à isoler les cristaux formés, procédé caractérisé en ce qu'on utilise le t.-butylméthyléther comme solvant.

**Claims**

1. Process for preparing (-)-[methyl (2*R*,3*S*)-2,3-epoxy-3-(4-methoxyphenyl)propionate], wherein a saturated solution is first prepared from 1 part by weight of *trans*-(±)-[methyl 2,3-epoxy-3-(4-methoxyphenyl)propionate] and from 0 to 5 parts by weight of (-)-[methyl (2*R*,3*S*)-2,3-epoxy-3-(4-methoxyphenyl)propionate], a crystalline seed of (-)-[methyl (2*R*,3*S*)-2,3-epoxy-3-(4-methoxyphenyl)propionate] is added to the solution, crystallization is allowed to take place and the crystals formed are isolated, characterised in that t.-butyl methylether is used as a solvent.

**Patentansprüche**

1. Verfahren zur Herstellung des (-)-(2*R*,3*S*)-2,3-Epoxy-3-(4-methoxyphenyl)propionsäuremethylesters, wobei man zunächst ausgehend von 1 Gew.-Teil *trans*-(±)-2,3-Epoxy-3-(4-methoxyphenyl)propionsäuremethylester und 0 bis 5 Gew.-Teilen (-)-(2*R*,3*S*)-2,3-Epoxy-3-(4-methoxyphenyl)propionsäuremethylester eine gesättigte Lösung herstellt, dann zu dieser Lösung einen kristallinen Keim des (-)-(2*R*,3*S*)-2,3-Epoxy-3-(4-methoxyphenyl)propionsäuremethylesters zusetzt, kristallisieren läßt und die gebildeten Kristalle isoliert, dadurch gekennzeichnet, daß man tert.-Butylmethylether als Lösungsmittel verwendet.

\*

$$\left(ee = \frac{[S] - [R]}{[S] + [R]} \times 100\%, [S]\ et\ [R]\ \text{étant les concentrations des deux énantiomères}\right)$$